(19) Europäisches Patentamt / European Patent Office / Office européen des brevets

(11) **EP 3 074 371 B1**

## (12) FASCICULE DE BREVET EUROPEEN

(45) Date de publication et mention
de la délivrance du brevet:
**25.04.2018 Bulletin 2018/17**

(21) Numéro de dépôt: **14805586.6**

(22) Date de dépôt: **28.11.2014**

(51) Int Cl.:
**C07C 7/10** *(2006.01)*  **C07C 11/18** *(2006.01)*
**C07C 11/167** *(2006.01)*

(86) Numéro de dépôt international:
**PCT/EP2014/076003**

(87) Numéro de publication internationale:
**WO 2015/079041 (04.06.2015 Gazette 2015/22)**

(54) **PROCÉDÉ D'EXTRACTION ET DE PURIFICATION DES DIOLÉFINES CONTENUES DANS UN MÉLANGE D'HYDROCARBURES**

VERFAHREN ZUR GEWINNUNG UND REINIGUNG VON DIOLEFINEN IN EINER MISCHUNG AUS KOHLENWASSERSTOFFEN

PROCESS FOR EXTRACTING AND PURIFYING DIOLEFINS CONTAINED IN A MIXTURE OF HYDROCARBONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.11.2013 FR 1361832**

(43) Date de publication de la demande:
**05.10.2016 Bulletin 2016/40**

(73) Titulaires:
- **IFP Energies nouvelles
  92500 Rueil-Malmaison (FR)**
- **Compagnie Générale des Etablissements Michelin
  63000 Clermont-Ferrand (FR)**

(72) Inventeurs:
- **DROZDZ, Sophie
  69126 Brindas (FR)**
- **JACQUIN, Marc
  69002 Lyon (FR)**

(56) Documents cités:
**FR-A5- 2 036 057     US-A- 3 328 480**

## EP 3 074 371 B1

**Description**

## DOMAINE TECHNIQUE DE L'INVENTION

La présente invention concerne un procédé d'extraction et de purification par solvant des dioléfines contenues dans des mélanges complexes. Plus particulièrement, l'invention concerne un procédé d'extraction et de purification par solvant du 1,3-butadiène d'un mélange d'hydrocarbures à 4 atomes de carbone ($C_4$), de l'isoprène d'un mélange d'hydrocarbures à 5 atomes de carbone ($C_5$) ou du 1,3-butadiène et de l'isoprène d'un mélange d'hydrocarbures en $C_4$-$C_5$. Lesdits mélanges d'hydrocarbures peuvent également contenir des impuretés oxygénées, azotées et soufrées ayant une température d'ébullition proche des composés que l'on cherche à séparer et/ou formant des azéotropes avec ces derniers.

De manière non limitative, ces dioléfines peuvent être produites par un procédé de vapocraquage d'une coupe pétrolière, un procédé de déshydrogénation d'oléfines et/ou de paraffines, ou bien encore un procédé du type Lebedev.

## ART ANTÉRIEUR

Il est pratiquement impossible de séparer les hydrocarbures saturés et insaturés de même nombre d'atomes de carbone par distillation simple étant donné les différences de volatilité extrêmement minimes entre ces hydrocarbures et à cause de la possible formation d'azéotropes entre certains d'entre eux.

Si l'on veut isoler un hydrocarbure particulier, par exemple le 1,3-butadiène d'une coupe $C_4$, ou l'isoprène d'une coupe $C_5$, ou le 1,3-butadiène et l'isoprène d'un mélange d'hydrocarbures en $C_4$-$C_5$, il faut adjoindre au mélange d'hydrocarbures un corps qui agit chimiquement, ou physiquement sur les espèces à séparer.

À titre d'exemple, la distillation extractive de la coupe $C_4$ issu du vapocraquage d'une coupe pétrolière est réalisée en présence d'un solvant inerte chimiquement vis à vis des espèces à séparer, mais ayant une affinité plus grande pour les composés insaturés. Pour être mis en oeuvre au sein d'un procédé de distillation extractive, ce solvant doit par ailleurs être plus lourd que les composés que l'on cherche à séparer et ne pas former d'azéotropes avec eux.

Dans une première colonne à distiller, le solvant est introduit en tête et la charge $C_4$ est introduite à un étage intermédiaire. Les composés $C_4$ saturés ou faiblement insaturés migrent vers la tête de la colonne, où ils sont partiellement condensés, la fraction condensée servant de reflux, alors que les composés $C_4$ fortement insaturés migrent vers le fond de la colonne avec le solvant. Le fond de la première colonne est envoyé vers une seconde colonne, dont les conditions de fonctionnement (pression, niveaux de température) sont différentes de la première. Cette seconde colonne permet de récupérer en tête les composés $C_4$ fortement insaturés et en fond le solvant régénéré, qui est renvoyé vers la première colonne.

Ces procédés de distillation extractive souffrent néanmoins de nombreux inconvénients. En effet, pour réaliser une bonne séparation et atteindre les spécifications demandées, le débit de solvant doit être très supérieure au débit de charge (typiquement un rapport 10), le nombre de plateaux doit être élevé (typiquement plus de 50 plateaux réels), et le taux de reflux (débit de reflux sur débit de charge) est important. Il s'en suit donc de lourds investissement et frais de fonctionnement. Par ailleurs, les solvants qui permettent de faire une telle séparation sont des solvants très toxiques, dont on cherche à limiter l'utilisation aujourd'hui, par exemple la N-MethylPyrrolidone (MNP) et la DiMethylFormamide (DMF).

Une alternative intéressante à ces procédés de distillation extractive est proposée dans le brevet FR 2,036,057. Au lieu de mettre à profit la différence de solubilité d'hydrocarbures $C_4$ et/ou $C_5$ en fonction du degré d'insaturation entre une phase liquide et une phase vapeur (comme c'est le cas en distillation extractive) pour réaliser la séparation, on met à profit la différence de solubilité en fonction du degré d'insaturation entre deux phases liquides.

Ce procédé met donc en oeuvre au moins deux solvants : un solvant polaire et un solvant apolaire. Ces solvants doivent avoir une faible solubilité mutuelle, et donc former deux phases lorsqu'il sont mélangés dans un ratio propice à la séparation des composés de la charge. Ces solvants doivent aussi avoir une différence de densité suffisante pour permettre une séparation des phases rapide au sein d'un décanteur, le solvant polaire étant a priori plus dense que le solvant apolaire.

Par ailleurs, le solvant apolaire doit avoir une température d'ébullition supérieure à celle de la charge, pour être facilement séparable par distillation des composés de la charge qui y sont dissout. Selon l'art antérieur, ce solvant apolaire est un hydrocarbure paraffinique ou cycloparaffinique ou un mélange d'hydrocarbures paraffiniques et cycloparaffiniques possédant de préférence 5 à 10 atomes de carbone par molécule. Selon une forme de réalisation préférée de l'art antérieur, il est constitué en majeure partie d'une coupe d'hydrocarbures paraffiniques et cycloparaffiniques à 5 et 6 atomes de carbone.

Enfin, le solvant polaire doit avoir une température d'ébullition supérieure à celle du solvant apolaire, et donc supérieure à celle de la charge. Selon l'art antérieur, ce solvant polaire peut être un dialkylsulfoxydes, la N-MéthylPyrrolidone (MNP), la butyrolactone, la DiMéthylFormamide (DMF), l'acétonitrile (ACN), purs ou contenant jusqu'à 20 %pds

2

d'eau. Selon une forme de réalisation préférée de l'art antérieur, le solvant polaire est du diméthylsulfoxyde (DMSO) commercial, auquel on peut ajouter jusqu'à 10 %pds d'eau.

Les caractéristiques générales que doit posséder le solvant polaire tel que décrit dans FR 2,036,057 ne sont cependant pas suffisamment précises pour permettre à un Homme du métier se basant sur le seul enseignement de ce texte de sélectionner un solvant performant.

Le document US 3,328,480 décrit la séparation des dioléfines contenues dans un effluent hydrocarbures par extraction liquide-liquide, mettant en oeuvre un solvant comprenant du propylène carbonate et de l'éthylène carbonate. Ce document divulgue que la proportion de propylène carbonate doit être majoritaire. Or, la demanderesse a découvert que des performances supérieures pouvaient être atteintes avec des proportions de propylène carbonate minoritaires.

Au sein du procédé selon l'art antérieur, la charge est envoyée dans un extracteur liquide-liquide constitué de deux zones, à savoir dans la partie supérieure une zone de lavage, et dans la partie inférieure une zone de contre-lavage, qui permettent de réaliser la séparation entre les composés les moins insaturés, typiquement les paraffines et les oléfines, et les composés les plus insaturés, typiquement les dioléfines et les aromatiques, de la charge.

La charge est introduite entre la zone de lavage et la zone de contre-lavage. En tête de la zone de lavage, on introduit le solvant polaire et l'on soutire le raffinat constitué du solvant apolaire et des constituants les moins insaturés de la charge. En fond de la zone de contre-lavage, on introduit le solvant apolaire et l'on soutire l'extrait constitué du solvant polaire et des constituants les plus insaturés de la charge.

La zone de lavage permet de garantir le rendement d'extraction des composés les plus insaturés, alors que la zone de contre-lavage permet de garantir leur pureté. Le raffinat est envoyé vers une colonne à distiller afin de récupérer les composés saturés et de régénérer le solvant apolaire, alors que l'extrait est envoyé vers un second extracteur liquide-liquide constitué d'une unique zone dite "de régénération". Dans cet extracteur liquide-liquide, on fait circuler le solvant apolaire à contre-courant du solvant polaire de manière à réextraire la totalité des composés les plus insaturés dans le solvant apolaire. Ce dernier est ensuite envoyé vers une colonne à distiller afin de récupérer les composés les plus insaturés et de régénérer le solvant apolaire. Le solvant polaire régénéré est renvoyé vers le premier extracteur liquide-liquide pour un nouveau cycle.

Un premier avantage de ce procédé selon l'art antérieur est qu'il permet la séparation des hydrocarbures par familles chimiques indépendamment de leur nombre de carbone, ce qui n'est pas possible par distillation extractive qui est limité à une coupe fine de distillation. Il est ainsi possible d'extraire non seulement le 1,3-butadiène d'un mélange d'hydrocarbures en $C_4$ ou l'isoprène d'un mélange d'hydrocarbures en $C_5$, mais également le 1,3-butadiène et l'isoprène conjointement, contenus dans un mélange d'hydrocarbures à 4 et 5 atomes de carbones.

Un second avantage du procédé est que l'extraction est réalisée à basse température ce qui diminue considérablement les risques de dégradation des produits instables comme les dioléfines.

Un autre avantage du procédé est qu'il permet d'accéder à de hautes puretés du produit final avec un rendement élevé, grâce à une circulation judicieusement choisie du solvant polaire ayant un rôle d'extraction et du solvant apolaire ayant un rôle de réextraction.

Cependant, les solvants décrits dans l'état de l'art imposent un taux de dilution de la charge dans les solvants polaires et apolaires important, avec un rapport poids en débit du solvant polaire d'extraction/débit de la charge compris entre 1 et 30 (soit le même ordre de grandeur qu'en distillation extractive), sous peine de dégrader la qualité de la séparation. Or, les débits pouvant circuler dans une colonne d'extraction liquide-liquide sont plus faibles que ceux pouvant circuler dans une colonne à distiller telle que celles utilisées en distillation extractive. Donc, pour traiter un même débit de charge, avec un même taux de dilution de la charge par rapport au(x) solvant(s), il faudra davantage de colonnes d'extraction liquide-liquide que de distillation extractive, ce qui augmente les coûts opératoires et les coûts d'investissement. Une diminution du taux de dilution, et donc de la qualité de la séparation, pourrait être compensée par une augmentation du nombre d'étage de séparation. Néanmoins, malgré les bonnes performances des solvants décrits dans l'état de l'art, le nombre d'étages théoriques nécessaire pour obtenir des dioléfines de haute pureté avec un bon rendement est important. La hauteur des extracteurs liquide-liquide devient donc vite rédhibitoire d'un point de vue technologique et il n'est plus guère possible, avec les technologies actuelles, d'augmenter ce nombre d'étages.

La demanderesse a découvert qu'une formulation particulière de la solution polaire absorbante permettait de résoudre plusieurs de ces inconvénients.

## OBJET ET INTÉRÊT DE L'INVENTION

L'invention concerne une solution absorbante comprenant au moins un solvant polaire choisi parmi les dialkylcarbonates cycliques.

L'invention concerne également un procédé d'extraction en phase liquide des dioléfines contenues dans une charge hydrocarbures de divers degrés de saturation.

De manière surprenante, la demanderesse a découvert que l'utilisation de solvants polaires différents de ceux décrits dans l'art antérieur permettaient d'obtenir des performances améliorées, en particulier par rapport au solvant préféré décrit dans FR 2.036.057 qui est le DMSO. En effet, la mise en oeuvre des solvants identifiés par la demanderesse dans un procédé tel que décrit dans le brevet FR 2,036,057 permettent une réduction significative du nombre d'étages théoriques pour réaliser une même séparation. Par ailleurs, ces solvants permettent d'envisager d'augmenter significativement le débit de charge par rapport aux débits de solvants polaires et apolaires, tout en maintenant une bonne qualité de séparation, ce qui est impossible avec les solvants revendiqués dans l'art antérieur.

En particulier, la demanderesse a découvert qu'un diakylcarbonate cyclique, par exemple l'éthylène carbonate, le propylène carbonate, ou un mélange d'éthylène carbonate et de propylène carbonate, était un excellent solvant polaire pour réaliser la séparation.

## DESCRIPTION DÉTAILLÉE DE L'INVENTION

L'invention concerne une solution absorbante comprenant au moins un solvant polaire choisi parmi les dialkylcarbonates cycliques.

De manière avantageuse, l'invention concerne une solution absorbante contenant pour seuls solvants polaires des solvants polaires choisis parmi l'éthylène carbonate et le propylène carbonate, pris seul ou en mélange.

Une variante préférée de l'invention est une solution absorbante contenant pour seuls solvants polaires un mélange d'éthylène carbonate (EC) et de propylène carbonate (PC) dans un rapport massique EC/PC compris entre 90/10 et 60/40, préférentiellement compris entre 75/25 et 65/35, saturée en solvants apolaires choisis parmi un hydrocarbure paraffinique ou cycloparaffinique ou un mélange d'hydrocarbures paraffiniques et cycloparaffiniques possédant de 5 à 10 atomes de carbone par molécule.

L'invention concerne également l'utilisation d'une solution absorbante telle que définie ci-dessus dans un procédé d'extraction en phase liquide des dioléfines contenues dans une charge hydrocarbures de divers degrés de saturation.

En outre, l'invention concerne un procédé d'extraction en phase liquide des dioléfines contenues dans une charge hydrocarbures de divers degrés de saturation comprenant au moins une étape d'extraction liquide-liquide à contre-courant alimentée par ladite charge hydrocarbures, et par une solution d'extraction, ladite solution d'extraction alimentée dans ladite étape a) étant une solution absorbante telle que définie ci-dessus.

Ainsi, l'invention concerne notamment un procédé d'extraction en phase liquide des dioléfines contenues dans une charge hydrocarbures de divers degrés de saturation comprenant au moins :

a) une étape d'extraction liquide-liquide à contre-courant alimentée :

- en au moins un point intermédiaire par ladite charge hydrocarbures,
- en tête par une solution d'extraction constituée de la solution polaire d'extraction issue de l'étape e) et du raffinat de réextraction issu de l'étape b),
- en fond par une solution auxiliaire d'extraction constituée d'une fraction de la solution apolaire auxiliaire issue de l'étape e) de décantation et/ou d'une fraction de l'extrait de réextraction soutiré en tête de la colonne d'extraction de l'étape b) de réextraction,

opérant à une température comprise entre 20 et 60°C, une pression comprise entre 0,1 et 1 MPa et un rapport poids en débit de solution d'extraction/débit de la charge hydrocarbures inférieur à 1 et produisant :

- en tête un raffinat principal, comprenant plus de 95% des hydrocarbures oléfiniques et paraffiniques compris dans ladite charge hydrocarbures,
- et en fond un extrait principal comprenant plus de 95% des hydrocarbures dioléfiniques compris dans ladite charge hydrocarbures,

b) une étape de réextraction liquide-liquide à contre-courant alimentée :

- en tête par ledit extrait principal issu de l'étape a),
- en fond par une fraction de la solution apolaire auxiliaire issue de l'étape e), opérant à une température comprise entre 20 et 60°C, une pression comprise entre 0,1 et 1 MPa, le rapport en poids débit de fraction de solution apolaire auxiliaire/débit d'extrait principal étant compris entre 0,1 et 1, et produisant :
- en tête un extrait de réextraction comprenant au moins 90% des hydrocarbures dioléfiniques compris dans ledit extrait principal,
- et en fond un raffinat de réextraction,

c) une étape de séparation des dioléfines dans laquelle au moins une fraction dudit extrait de réextraction issu de l'étape b) est alimentée en au moins un point intermédiaire d'une colonne à distiller, un effluent dioléfines est soutiré en tête de ladite colonne, et un résidu de séparation des dioléfines est soutiré en fond de ladite colonne,

d) une étape de séparation des hydrocarbures dans laquelle ledit raffinat principal issu de l'étape a) est alimenté en au moins un point intermédiaire d'une colonne à distiller, un effluent hydrocarbures est soutiré en tête de ladite colonne, et un résidu de séparation des hydrocarbures est soutiré en fond de ladite colonne,

e) une étape de décantation alimentée par ledit résidu de séparation des dioléfines issu de l'étape c) et ledit résidu de séparation des hydrocarbures issu de l'étape d), permettant la séparation d'une solution polaire d'extraction et d'une solution apolaire auxiliaire, ladite solution polaire d'extraction étant ensuite recyclée vers l'étape a), ladite solution apolaire auxiliaire étant ensuite recyclée vers l'étape b) et/ou l'étape a),

ladite solution d'extraction alimentée dans ladite étape a) étant une solution absorbante comprenant au moins un solvant polaire choisi parmi les dialkylcarbonates cycliques, de manière avantageuse, une solution absorbante contenant pour seuls solvants polaires des solvants polaires choisis parmi l'éthylène carbonate et le propylène carbonate, pris seul ou en mélange, et de manière préférée une solution absorbante contenant pour seuls solvants polaires un mélange d'éthylène carbonate (EC) et de propylène carbonate (PC) dans un rapport massique EC/PC compris entre 90/10 et 60/40, préférentiellement compris entre 75/25 et 65/35, saturée en solvants apolaires choisis parmi un hydrocarbure paraffinique ou cycloparaffinique ou un mélange d'hydrocarbures paraffiniques et cycloparaffiniques possédant de 5 à 10 atomes de carbone par molécule.

Ladite charge hydrocarbures contient des composés hydrocarbonés de divers degrés de saturation ayant de 1 à 10 atomes de carbone, préférentiellement de 4 à 7 atomes de carbone, et de manière préférée de 4 à 5 atomes de carbone. Par divers degrés de saturation, on entend que ladite charge hydrocarbures comprend des paraffines, des oléfines, des dioléfines, des composés polyinsaturés, la teneur en lesdites dioléfines étant de préférence comprise entre 20 et 99,5% poids. En particulier, ladite charge hydrocarbures comprend du 1,3-butadiène et/ou de l'isoprène.

## Étape a) d'extraction

Conformément à l'invention, dans une étape a) d'extraction, une colonne d'extraction liquide-liquide à contre-courant est alimentée en au moins un point intermédiaire, préférentiellement en un point intermédiaire, par ladite charge hydrocarbures, en tête par une solution d'extraction constituée de la solution polaire d'extraction issue de l'étape e) et du raffinat de réextraction issu de l'étape b), et en fond par une solution auxiliaire d'extraction constituée d'une fraction de la solution apolaire auxiliaire issue de l'étape e) de décantation et/ou d'une fraction de l'extrait de réextraction soutiré en tête de la colonne d'extraction de l'étape b) de réextraction.

On soutire en tête un raffinat principal, lequel alimente ensuite l'étape d) de séparation des hydrocarbures, et en fond un extrait principal qui est recyclé vers l'étape b) de réextraction.

Par point intermédiaire, on entend que ladite charge hydrocarbures est alimentée par un plateau d'alimentation qui est situé entre le plateau de tête et le plateau de fond de ladite colonne d'extraction.

Ladite étape a) d'extraction est opérée à une température comprise entre 20 et 60°C, à une pression comprise entre 0,1 et 1 MPa. Le rapport en poids débit de solution d'extraction/débit de la charge hydrocarbures est inférieur à 1.

Ladite solution d'extraction comprend au moins un solvant polaire choisi parmi les dialkylcarbonates cycliques.

De manière préférée, Ladite solution d'extraction contient pour seuls solvants polaires des solvants polaires choisis parmi les dialkylcarbonates cycliques, préférentiellement parmi l'éthylène carbonate et le propylène carbonate, pris seul ou en mélange. De manière préférée, ladite solution d'extraction contient comme seuls solvants polaires un mélange d'éthylène carbonate et de propylène carbonate contenant moins de 50% poids, de manière préférée entre 10 et 40% poids, et préférentiellement entre 25% et 35% poids de propylène carbonate, le pourcentage poids étant exprimé par rapport à la masse de solvants polaires dans ladite solution d'extraction.

Ladite solution auxiliaire d'extraction comprend au moins un solvant apolaire choisi parmi les solvants apolaires connus de l'Homme du métier, c'est à dire choisi parmi un hydrocarbure paraffinique ou cycloparaffinique ou un mélange d'hydrocarbures paraffiniques et cycloparaffiniques possédant de préférence 5 à 10 atomes de carbone par molécule. De manière préférée, ledit solvant apolaire est une coupe d'hydrocarbures paraffiniques et cycloparaffiniques à 5 et 6 atomes de carbone, et de manière très préféré ledit solvant apolaire est le cyclohexane.

Les pertes en solvant polaires et apolaires dans les différents effluents du procédé sont infimes. Les différents solvants tournent donc en rond dans le procédé selon l'invention. Un appoint en solvant polaire peut toutefois être réalisé au niveau de l'alimentation en solution d'extraction, tout comme un appoint en solvant apolaire peut être réalisé au niveau de l'alimentation en solution auxiliaire d'extraction.

L'éthylène carbonate a un pouvoir de séparation supérieur à celui du propylène carbonate. Néanmoins, l'éthylène carbonate ayant une température de fusion de 38°C, il ne peut être utilisé pur que si la température des zones d'extraction des étapes a) et b) est supérieure à cette température de fusion, préférentiellement supérieure à 40°C.

La demanderesse a découvert que l'ajout de 10 à 40% poids, et préférentiellement de 25% à 35% poids de propylène carbonate permet de ramener la température de fusion du mélange éthylène carbonate/propylène carbonate en dessous de la température ambiante (c'est à dire moins de 20°C), tandis que le pouvoir de séparation du mélange est sensiblement le même que celui de l'éthylène carbonate.

De part la différence de densité, ladite solution auxiliaire d'extraction se déplace à contre courant de ladite solution d'extraction ainsi que des hydrocarbures extraits sélectivement de la charge hydrocarbures. Elle réextrait les hydrocarbures oléfiniques et paraffiniques entraînés avec le solvant polaire.

Ledit raffinat principal comprend plus de 95 %, préférentiellement plus de 99 %, et de manière préférée la totalité des hydrocarbures paraffiniques et oléfiniques contenus dans ladite charge hydrocarbure.

Ledit extrait principal comprend plus de 95 %, préférentiellement plus de 99 %, et de manière préférée la totalité des hydrocarbures dioléfiniques contenus dans ladite charge hydrocarbure.

**Étape b) de réextraction**

Conformément à l'invention, dans une étape b) de réextraction, une colonne d'extraction liquide-liquide à contre-courant est alimentée en tête par l'extrait principal issu de l'étape a) et en fond par une fraction de la solution apolaire auxiliaire issue de l'étape e).

On soutire en tête un extrait de réextraction, lequel alimente une étape c) de séparation des hydrocarbures, et en fond un raffinat de réextraction qui est recyclé vers l'étape a) d'extraction.

Ladite étape b) est opérée à une température comprise entre 20 et 60°C, à une pression comprise entre 0,1 et 1 MPa. Le débit de solution apolaire auxiliaire alimentant ladite étape b) est fonction du débit d'extrait principal issu de l'étape a) d'extraction, ce débit étant calculé de manière à réextraire le maximum de diènes contenus dans l'extrait principal. Le rapport en poids débit de fraction de solution apolaire auxiliaire/débit d'extrait principal est compris entre 0,1 et 1.

Dans le cas où la solution auxiliaire d'extraction comprend au moins une fraction de l'extrait de réextraction soutiré en tête de la colonne d'extraction de l'étape b) de réextraction, ladite fraction peut représenter jusqu'à 80% du débit total d'extrait de réextraction soutiré en tête de la colonne d'extraction de ladite étape b).

Le débit de la fraction de solution apolaire auxiliaire est choisi de manière à avoir une réextraction totale des dioléfines. Par réextraction totale, on entend qu'au moins 90% des dioléfines contenues dans l'extrait principal, de préférence 95%, de manière très préférée 99% sont réextraites dans l'extrait de réextraction.

Ledit raffinat de réextraction comprend le solvant polaire extrait dudit extrait principal. Il est recyclé, en mélange avec la solution polaire d'extraction issue de l'étape e), vers l'étape a) d'extraction.

**Étape c) de séparation des dioléfines**

Conformément à l'invention, au moins une fraction dudit extrait de réextraction issu de l'étape b) alimente, dans une étape c) de séparation des dioléfines, une colonne à distiller en au moins un point intermédiaire, préférentiellement en un point intermédiaire.

Par point intermédiaire, on entend que ledit extrait de deuxième extraction est alimenté par un plateau d'alimentation qui est situé entre le plateau de tête et le plateau de fond de ladite colonne à distiller.

Ladite étape c) opère de préférence à une pression comprise entre 0,01 et 0,5 MPa, et de préférence à une pression inférieure à la pression d'opération de la colonne à distiller mise en oeuvre dans l'étape d) de manière à limiter la dégradation des dioléfines.

Un effluent dioléfines est soutiré en tête de ladite colonne à distiller. Il comprend au moins 98% des dioléfines contenues dans ledit extrait de réextraction, préférentiellement au moins 99%.

Un résidu de séparation des dioléfines est soutiré en fond de ladite colonne à distiller. Il comprend au moins 95% des solvants polaires et apolaires contenus dans ledit extrait de réextraction, préférentiellement au moins 99%. Ledit résidu alimente l'étape e) de décantation.

**Étape d) de séparation des hydrocarbures**

Conformément à l'invention, ledit raffinat principal issu de l'étape a) alimente, dans une étape d) de séparation des hydrocarbures, une colonne à distiller en au moins un point intermédiaire, préférentiellement en un point intermédiaire.

Par point intermédiaire, on entend que ledit raffinat principal est alimenté par un plateau d'alimentation qui est situé entre le plateau de tête et le plateau de fond de ladite colonne à distiller.

Ladite étape d) opère de préférence à une pression comprise entre 0,1 et 1 MPa.

Un effluent hydrocarbures est soutiré en tête de ladite colonne à distiller. Il comprend au moins 95% des hydrocarbures contenus dans ledit raffinat principal, préférentiellement au moins 99%.

Un résidu de séparation des hydrocarbures est soutiré en fond de ladite colonne à distiller. Il comprend au moins 95% des solvants polaires et apolaires contenus dans ledit raffinat principal, préférentiellement au moins 99%. Ledit résidu alimente l'étape e) de décantation.

### Étape e) de décantation

Conformément à l'invention, une étape e) de décantation est alimentée par ledit résidu de séparation des dioléfines issu de l'étape d) et par ledit résidu de séparation des hydrocarbures issu de l'étape d). Les solvants polaires et apolaires n'étant pas miscibles, ladite étape e) permet la séparation d'une solution polaire d'extraction et d'une solution apolaire auxiliaire comprenant respectivement les solvants polaires et les solvants apolaires.

Ladite étape e) de décantation est de préférence opérée à une température comprise entre 20 et 60°C et à une pression comprise entre 0,1 et 1 MPa.

Ladite solution polaire d'extraction est recyclée vers l'étape a) d'extraction.

Une fraction de ladite solution apolaire auxiliaire est recyclée vers l'étape b) de réextraction. La fraction restante est recyclée vers l'étape a) d'extraction.

### DESCRIPTION DES FIGURES

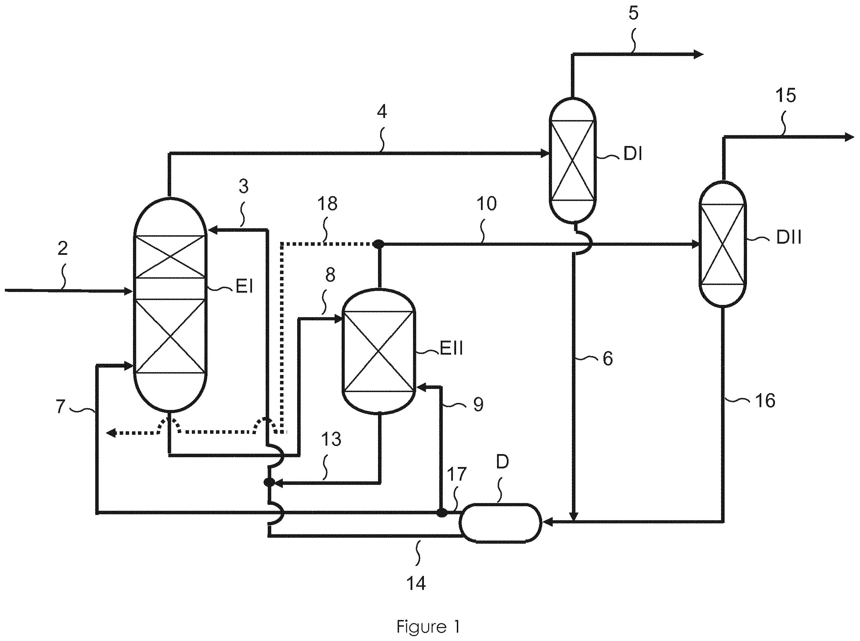

La figure 1 présente de manière non limitative un arrangement général du procédé selon l'invention. Une charge hydrocarbures (2) alimente en un point intermédiaire une colonne d'extraction liquide-liquide à contre-courant (EI). Cette dernière est alimentée en tête par une solution d'extraction (3) et en fond par une solution auxiliaire d'extraction (7), ladite solution auxiliaire d'extraction étant constituée d'une fraction de la solution apolaire auxiliaire (17) issue de (D) et/ou d'une fraction (18) de l'extrait de réextraction.

Un raffinat principal (4) comprenant le solvant apolaire et les hydrocarbures oléfiniques et paraffiniques est soutiré en tête de ladite colonne d'extraction (EI). Il est séparé dans une colonne à distiller (DI) en un résidu de séparation des hydrocarbures (6) comprenant le solvant apolaire saturé en solvant polaire et en un effluent hydrocarbures (5).

Ledit résidu de séparation des hydrocarbures (6) est envoyé vers le décanteur (D), en mélange avec le résidu de séparation des dioléfines (16) de manière à être séparé par décantation en une solution apolaire auxiliaire (17) et une solution polaire d'extraction (14).

Une fraction (9) de ladite solution apolaire auxiliaire (17), appelée solution auxiliaire de réextraction, alimente une colonne de réextraction liquide-liquide (EII), cette dernière étant également alimenté en tête par l'extrait principal (8) soutiré en fond de la colonne d'extraction liquide-liquide (EI).

On soutire en tête de ladite colonne de réextraction (EII) un extrait de réextraction (10) comprenant les dioléfines, et en fond un raffinat de réextraction (13) comprenant du solvant polaire, ledit raffinat de réextraction étant envoyé, en mélange avec la solution polaire d'extraction (14) issue du décanteur (D) en tête de la colonne de première extraction (EI).

L'extrait de réextraction (10) alimente en un point intermédiaire une colonne à distiller (DII) pour être séparé en un effluent dioléfines (15) et en un résidu de séparation des dioléfines (16), ce dernier alimentant ensuite le décanteur (D). Une fraction dudit extrait de réextraction (18) peut alimenter le fond la colonne d'extraction (EI).

### EXEMPLES

### Exemple 1

*Dans cet exemple, on compare les performances obtenues avec un solvant conforme à l'invention à celles obtenues avec les solvants décrits dans l'art antérieur pour un taux de dilution donné.*

On analyse les performances obtenues pour séparer l'isoprène d'une charge hydrocarbures contenant 30%poids d'isoprène (dioléfine en $C_5$) et 70% poids de 2-méthyl-1-butène (oléfine en $C_5$).

On mesure le coefficient de partage K de l'isoprène, noté A, et du 2-méthyl-1-butène, noté B, entre un solvant apolaire (ici le cyclohexane) et différents solvants polaires testés. Les coefficients de partage K sont ici mesurés en solution infiniment diluée, de manière à être représentatif des forts taux de dilution de la charge dans les solvants qui sont décrits dans l'art antérieur.

On note $K_A = [A]_{S1}/[A]_{S2}$ et $K_B = [B]_{S1}/[B]_{S2}$, où $[\ ]_{S1}$ est une concentration en mol/kg dans le solvant polaire, noté S1, et $[\ ]_{S2}$ est une concentration en mol/kg dans le solvant apolaire, noté S2.

On définit également la sélectivité d'absorption de A par rapport à B $S_{A/B} = K_A/K_B$.

Le solvant polaire conforme à l'invention contient 75% poids d'éthylène carbonate et 25% poids de propylène carbonate.

On teste les solvants cités dans FR 2.036.057, soit le DMSO, l'acétonitrile (ACN, le diméthylformamide (DMF), la n-méthylpyrolidone (NMP), le butyrolactone. On teste également des solvants correspondant aux critères énumérés dans FR 2.036.057, c'est à dire ayant une densité supérieure à la charge hydrocarbures, dont la température d'ébullition est très supérieure à la charge et supérieure à celle du solvant apolaire, et faiblement soluble dans le solvant apolaire, comme le triméthyl phosphate, le 2-cyanoethyl ether et un mélange comportant 75% poids de sulfolane et 25% poids d'acétone.

$K_A$, $K_B$ et $S_{A/B}$ sont donnés dans le tableau ci-dessous pour différents solvants polaires, selon l'invention et selon l'art antérieur.

Tableau 1 - mesures à 20°C

| Solvant polaire | $K_A$ | $K_B$ | $S_{A/B}$ |
|---|---|---|---|
| EC 90%pds - PC 10%pds (conforme) | 0,07 | 0,02 | 3,1 |
| EC 75%pds - PC 25%pds (conforme) | 0,07 | 0,02 | 3,1 |
| EC 70%pds - PC 30%pds (conforme) | 0,08 | 0,03 | 3,2 |
| EC 60%pds - PC 40%pds (conforme) | 0,08 | 0,03 | 2,9 |
| EC 40%pds - PC 60%pds | 0,12 | 0,05 | 2.5 |
| EC 30%pds - PC 70%pds | 0,13 | 0,05 | 2.5 |
| EC 20%pds - PC 80%pds | 0,14 | 0,06 | 2.4 |
| DMSO | 0,16 | 0,06 | 2,8 |
| ACN | 0,64 | 0,34 | 1,9 |
| NMP | 0,67 | 0,47 | 1,4 |
| DMF | 0,42 | 0,36 | 1,1 |
| butyrolactone | 0,27 | 0,11 | 2,5 |
| Triméthyl phosphate | 0,22 | 0,10 | 2,2 |
| 2-cyanoethyl ether | 0,08 | 0,05 | 1,7 |
| sulfolane 75% pds - acétone 25% pds | 0,21 | 0,09 | 2,3 |

Tableau 2 - mesures à 40°C

| Solvant polaire | $K_A$ | $K_B$ | $S_{A/B}$ |
|---|---|---|---|
| EC 90%pds - PC 10%pds (conforme) | 0,07 | 0,02 | 3,0 |
| EC 75%pds - PC 25%pds (conforme) | 0,09 | 0,03 | 2,8 |
| DMSO | 0,17 | 0,07 | 2,6 |

On peut voir avec ces tableaux que le solvant selon l'invention offre une meilleure sélectivité $S_{A/B}$, mais une plus faible capacité d'absorption $K_A$ que les solvants de l'art antérieur.

Le procédé d'extraction des dioléfines est opéré avec chacun de ces solvants en fixant les paramètres suivant :

- pureté de l'isoprène obtenu : 99,5% poids

- rendement global d'extraction de l'isoprène : 97% (isoprène produit/isoprène dans la charge hydrocarbures)

- taux de dilution dans l'extracteur (EI) fixé à 1 pour 10, c'est à dire que pour 1 kg/h de charge F rentrant dans l'extracteur (EI), le débit total (solution d'extraction + solution auxiliaire d'extraction) est de 10 kg/h.

- nombre d'étages théoriques dans la colonne de réextraction (EII)=5

On détermine pour chaque solvant polaire le nombre d'étages théoriques nécessaires dans la colonne d'extraction (EI), ainsi que les débits de solvants polaire Q1 alimentant (EI) et apolaire Qa1 alimentant (EI) et Qa2 (EII). Les

résultats sont indiqués dans le tableau 2.

Le tableau ci-dessous donne le nombre d'étages théoriques minimum NET nécessaires pour réaliser la séparation et les débits de solvants associés.

Tableau 3 - Extraction liquide-liquide opérée à 20°C

|  | NET (EI) | Q1 (EI) | Qa1 (EI) | Qa2 (EII) | Total solvant apolaire Qa1 + Qa2 |
|---|---|---|---|---|---|
| EC 90%pds - PC 10%pds (conforme) | 16 | 9,6 | 0,4 | 1,5 | 1,9 |
| EC 75%pds - PC 25%pds (conforme) | 15 | 9,6 | 0,4 | 1,5 | 1,9 |
| EC 70%pds - PC 30%pds (conforme) | 15 | 9,5 | 0,5 | 1,8 | 2,3 |
| EC 60%pds - PC 40%pds (conforme) | 17 | 9,5 | 0,5 | 1,8 | 2,3 |
| EC 40%pds - PC 60%pds | 20 | 9.1 | 0.9 | 2.4 | 3.3 |
| EC 30%pds - PC 70%pds | 20 | 9.2 | 0.8 | 2.6 | 3.4 |
| EC 20%pds - PC 80%pds | 20 | 9.1 | 0.9 | 2.9 | 3.8 |
| DMSO | 17 | 9,1 | 0,9 | 3,2 | 4,1 |
| ACN | 28 | 6,7 | 3,3 | 9,6 | 12,9 |
| NMP | 50 | 6,3 | 3,7 | 9,5 | 13,2 |
| DMF | 128 | 7,2 | 2,8 | 6,6 | 9.4 |
| butyrolactone | 20 | 8,5 | 1,5 | 5,1 | 6,6 |
| Triméthyl phosphate | 22 | 8,6 | 1,4 | 4,3 | 5,7 |
| 2-cyanoethyl ether | 35 | 9,4 | 0,6 | 1,7 | 2,3 |
| sulfolane 75% pds-acetone 25%pds | 22 | 8,7 | 1,3 | 4,1 | 5,4 |

Tableau 4 - Extraction liquide-liquide opérée à 40°C

|  | NET (EI) | Q1 (EI) | Qa1 (EI) | Qa2 (EII) | Total solvant apolaire Qa1 + Qa2 |
|---|---|---|---|---|---|
| EC 90%pds - PC 10%pds (conforme) | 16 | 9,6 | 0,4 | 1,4 | 1,8 |
| EC 75%pds - PC 25%pds (conforme) | 18 | 9,5 | 0,5 | 1,8 | 2,3 |
| DMSO | 19 | 9,0 | 1 | 3,4 | 4,4 |

On peut voir que la formulation conforme à l'invention affiche de très bonnes performances, meilleures que celles du solvant préféré de l'art antérieur, et bien meilleures que les autres solvants selon l'art antérieur. Le nombre d'étage théoriques nécessaires pour réaliser les performances fixées est plus faible avec le solvant conforme à l'invention qu'avec les solvants de l'art antérieur.

De plus, la somme des débits de solvant apolaire circulant dans l'unité Qa1+Qa2 en utilisant le solvant selon l'invention est réduite de 42% par rapport à l'utilisation du solvant préféré de l'art antérieur. Dans le procédé d'extraction, seul le solvant apolaire est régénéré par distillation. Moins de solvant apolaire signifie des colonnes à distiller plus petites et moins consommatrices d'énergie. La diminution des débits de solvants apolaires engendre donc une baisse significative des coûts opératoires et d'investissements.

**Exemple 2**

*Dans cet exemple, on compare les performances obtenues avec un solvant conforme à l'invention à celles obtenues avec les solvants décrits dans l'art antérieur pour différents taux de dilution.*

Pour illustrer les performances améliorées du solvant polaire conforme à l'invention par rapport au solvant polaire préféré de l'art antérieur, on mesure les coefficients de partage de l'isoprène, noté A, et du 2-méthyl-1-butène, noté B, entre un solvant apolaire (ici le cyclohexane) et une solution d'extraction conforme à l'invention d'une part, contenant contient 75% poids d'éthylène carbonate et 25% poids de propylène carbonate, et le solvant polaire préféré de l'art antérieur d'autre part (DMSO). On s'intéresse ensuite à la sélectivité d'absorption $S_{A/B}$ de A par rapport à B.

Contrairement à l'exemple précédent, les coefficients de partage K, et donc la sélectivité $S_{A/B}$, ne sont pas mesurés en solution infiniment dilué pour tenir compte de l'impact du taux de dilution de la charge par rapport aux solvants sur les performances du solvant polaire en terme de séparation. En effet, pour réduire les coûts associés à la séparation, on va chercher à réduire le taux de dilution, en injectant plus de charge pour un même débit de solvants.

On définit le taux dilution $\tau$ comme :

$$\tau = \frac{m_{\text{solvant apolaire}}}{m_{\text{charge}} + m_{\text{solvant apolaire}}}$$

où $m_{\text{charge}}$ est ici le débit de composés $C_5$ traités dans la charge hydrocarbures, et $m_{\text{solvant\_apolaire}}$ le débit de solvant apolaire. Les mesures de coefficient de partage ont été réalisées à des valeurs de taux de dilution $\tau$ de 0,05, 0,5 et 0,95.

Par ailleurs, les coefficients de partage K, et donc la sélectivité $S_{A/B}$, ont été mesurés en faisant varier la proportion des constituants A et B que l'on cherche à séparer. En effet, cette proportion varie tout au long de l'extracteur (EI), ce qui peut avoir un impact non négligeable sur les performances du solvant polaire en terme de séparation.

On définit la fraction massique $y_A$ du constituant A comme :

$$y_A = \frac{m_A}{m_A + m_B}$$

où $m_A$ et $m_B$ sont les masses de A et B. Les mesures de coefficient de partage K, et donc la sélectivité $S_{A/B}$, ont été réalisées à des valeurs $y_A$ comprises entre 0,98 et 0,02. Seules les valeurs extrêmes seront commentées ici. Elles correspondent aux sélectivités sur le premier et le dernier plateau d'un extracteur liquide-liquide qui sépare les constituants A et B avec une pureté de A de 98% et un rendement d'extraction de A de 98%.

Le tableau 5 regroupe les données expérimentales obtenues. De manière générale, quelque soit le solvant polaire utilisé, on voit que la diminution du taux de dilution entraine une diminution de la sélectivité d'absorption $S_{A/B}$ et ce, que l'on soit dans la zone riche en A ou dans la zone riche en B.

Dans la zone riche en B (zone garantissant le rendement d'extraction de A), les variations de sélectivité d'absorption $S_{A/B}$ avec le taux de dilution sont relativement faibles (environ 10%) quelque soit le solvant polaire utilisé, et les valeurs absolues de sélectivité d'absorption $S_{A/B}$ sont supérieures à 2,5. La séparation des constituants A et B peut donc être réalisée dans cette zone avec un nombre de plateaux raisonnables.

Dans la zone riche en A, qui garanti la pureté de A extrait, les variations de sélectivité d'absorption $S_{A/B}$ avec le taux de dilution sont importantes quelque soit le solvant polaire utilisé. Dans le cas du DMSO, la sélectivité d'absorption $S_{A/B}$ chute rapidement en dessous de 2,5. La sélectivité d'absorption de la solution d'extraction conforme $S_{A/B}$ reste au dessus de 2,5 même pour le taux de dilution le plus faible.

Tableau 5

| Taux de dilution | 0,95 | 0,5 | 0,05 |
|---|---|---|---|
| $S_{A/B}$ dans la zone de riche en A ($y_A$=0,98) | | | |
| Solvant conforme | 3,1 | 2,8 | 2,5 |
| DMSO | 2,8 | 2,3 | 1,3 |
| $S_{A/B}$ dans la zone de riche en B ($y_A$=0,02) | | | |
| Solvant conforme | 3,1 | 3,0 | 2,7 |
| DMSO | 2,8 | 2,5 | 2,6 |

Cet exemple comparatif illustre bien l'intérêt d'utiliser une solution d'extraction constituée de 75%pds d'éthylène carbonate et de 25%pds de propylène carbonate au lieu du DMSO.

Dans le cas du DMSO, le taux de dilution doit être maintenu élevé pour conserver une sélectivité d'absorption $S_{A/B}$ supérieure à 2,5 et garantir ainsi une forte pureté du produit A extrait. Dans le cas de la solution d'extraction conforme à l'invention, le taux de dilution peut être considérablement réduit, tout en maintenant de bonnes performances en terme de séparation des produits A et B. Ceci permet non seulement de réduire les coûts d'investissement (extracteur liquide-liquide et colonnes à distiller de plus faible diamètre, toutes choses égales par ailleurs) mais aussi les coûts opératoires (les débits de solvants à distiller rapporté à la charge étant plus faibles).

On notera également que les critères indiqués dans l'art antérieur ne permettent pas d'aboutir au solvant selon l'invention, car des solvants répondant à ces critères, non explicitement décrits dans l'art antérieur, présentent des performances inférieures au solvant conforme à l'invention.

**Revendications**

1. Solution absorbante contenant pour seuls solvants polaires un mélange d'éthylène carbonate (EC) et de propylène carbonate (PC) dans un rapport massique EC/PC compris entre 90/10 et 60/40, saturée en solvants apolaires choisis parmi un hydrocarbure paraffinique ou cycloparaffinique ou un mélange d'hydrocarbures paraffiniques et cycloparaffiniques possédant de 5 à 10 atomes de carbone par molécule.

2. Solution absorbante selon la revendication 1 dans laquelle le rapport massique EC/PC compris entre 75/25 et 65/35.

3. Solution absorbante selon l'une quelconque des revendications 1 ou 2, dans laquelle ledit solvant apolaire est une coupe d'hydrocarbures paraffiniques et cycloparaffiniques à 5 et 6 atomes de carbone, et de préférence le cyclohexane .

4. Utilisation d'une solution absorbante selon l'une quelconque des revendications 1 à 3, dans un procédé d'extraction en phase liquide des dioléfines contenues dans une charge hydrocarbures de divers degrés de saturation.

5. Procédé d'extraction en phase liquide des dioléfines contenues dans une charge hydrocarbures de divers degrés de saturation comprenant au moins une étape d'extraction liquide-liquide à contre-courant alimentée par ladite charge hydrocarbures, et par une solution d'extraction, ladite solution d'extraction étant une solution absorbante telle que définie selon l'une des revendications 1 à 3.

6. Procédé selon la revendication 5 comprenant au moins :

   a) une étape d'extraction liquide-liquide à contre-courant alimentée :

      - en au moins un point intermédiaire par ladite charge hydrocarbures,
      - en tête par une solution d'extraction constituée de la solution polaire d'extraction issue de l'étape e) et du raffinat de réextraction issu de l'étape b),
      - en fond par une solution auxiliaire d'extraction constituée d'une fraction de la solution apolaire auxiliaire issue de l'étape e) de décantation et/ou d'une fraction de l'extrait de réextraction soutiré en tête de la colonne d'extraction de l'étape b) de réextraction,

   opérant à une température comprise entre 20 et 60°C, une pression comprise entre 0,1 et 1 MPa et un rapport poids en débit de solution d'extraction/débit de la charge hydrocarbures inférieur à 1 et produisant :

      - en tête un raffinat principal, comprenant plus de 95% des hydrocarbures oléfiniques et paraffiniques compris dans ladite charge hydrocarbures,
      - et en fond un extrait principal comprenant plus de 95% des hydrocarbures dioléfiniques compris dans ladite charge hydrocarbures,

   b) une étape de réextraction liquide-liquide à contre-courant alimentée :

      - en tête par ledit extrait principal issu de l'étape a),
      - en fond par une fraction de la solution apolaire auxiliaire issue de l'étape e),

   opérant à une température comprise entre 20 et 60°C, une pression comprise entre 0,1 et 1 MPa, le rapport en poids débit de fraction de solution apolaire auxiliaire/débit d'extrait principal étant compris entre 0,1 et 1, et produisant :

      - en tête un extrait de réextraction comprenant au moins 90% des hydrocarbures dioléfiniques compris dans ledit extrait principal,
      - et en fond un raffinat de réextraction,

c) une étape de séparation des dioléfines dans laquelle au moins une fraction dudit extrait de réextraction issu de l'étape b) est alimentée en au moins un point intermédiaire d'une colonne à distiller, un effluent dioléfines est soutiré en tête de ladite colonne, et un résidu de séparation des dioléfines est soutiré en fond de ladite colonne,

d) une étape de séparation des hydrocarbures dans laquelle ledit raffinat principal issu de l'étape a) est alimenté en au moins un point intermédiaire d'une colonne à distiller, un effluent hydrocarbures est soutiré en tête de ladite colonne, et un résidu de séparation des hydrocarbures est soutirée en fond de ladite colonne,

e) une étape de décantation alimentée par ledit résidu de séparation des dioléfines issu de l'étape c) et ledit résidu de séparation des hydrocarbures issu de l'étape d), permettant la séparation d'une solution polaire d'extraction et d'une solution apolaire auxiliaire, ladite solution polaire d'extraction étant ensuite recyclée vers l'étape a), ladite solution apolaire auxiliaire étant ensuite recyclée vers l'étape b) et/ou l'étape a),

ladite solution d'extraction alimentée dans ladite étape a) étant une solution absorbante telle que définie selon l'une des revendications 1 à 3.

7. Procédé selon la revendication 6 dans lequel ladite solution auxiliaire d'extraction alimentant ladite étape a) d'extraction est constituée d'une fraction de ladite solution apolaire auxiliaire issue de ladite étape e).

8. Procédé selon l'une quelconque des revendications 6 à 7 dans lequel ladite solution auxiliaire d'extraction alimentant ladite étape a) d'extraction est constituée d'une fraction dudit extrait de réextraction issu de l'étape b).

9. Procédé selon l'une quelconque des revendications 5 à 8 dans lequel ladite charge hydrocarbures comprend de 20 à 99,5% poids de dioléfines.

10. Procédé selon l'une quelconque des revendications 6 à 9 dans lequel ledit raffinat principal comprend plus de 99% des hydrocarbures oléfiniques et paraffiniques compris dans ladite charge hydrocarbures.

11. Procédé selon l'une quelconque des revendications 6 à 10 dans lequel ledit extrait principal comprend plus de 99% des hydrocarbures dioléfiniques compris dans ladite charge hydrocarbures.

12. Procédé selon l'une quelconque des revendications 6 à 11 dans lequel ladite étape c) opère à une pression inférieure à la pression d'opération de la colonne mise en oeuvre dans ladite étape d).

13. Procédé selon l'une quelconque des revendications 6 à 12 dans lequel ladite étape d) opère à une pression comprise entre 0,1 et 1 MPa.

14. Procédé selon l'une quelconque des revendications 6 à 13 dans lequel ladite étape e) opère à une température comprise entre 20 et 60°C et une pression comprise entre 0,1 et 1 MPa.

**Patentansprüche**

1. Absorbierende Lösung, enthaltend als ausschließliche polare Lösungsmittel eine Mischung aus Ethylencarbonat (EC) und Propylencarbonat (PC) in einem Massenverhältnis EC/PC zwischen 90/10 und 60/40, gesättigt mit apolaren Lösungsmitteln, ausgewählt aus einem Paraffin- oder Cycloparaffin-Kohlenwasserstoff oder einer Mischung aus Paraffin- und Cycloparaffin-Kohlenwasserstoffen mit 5 bis 10 Kohlenstoffatomen pro Molekül.

2. Absorbierende Lösung nach Anspruch 1, wobei das Massenverhältnis EC/PC zwischen 75/25 und 65/35 liegt.

3. Absorbierende Lösung nach einem der Ansprüche 1 oder 2, wobei das apolare Lösungsmittel eine Paraffin- oder Cycloparaffin-Kohlenwasserstofffraktion mit 5 bis 6 Kohlenstoffatomen, und vorzugsweise Cyclohexan, ist.

4. Verwendung einer absorbierenden Lösung nach einem der Ansprüche 1 bis 3, in einem Verfahren zur Extraktion von Diolefinen in der Flüssigphase, die in einer Kohlenwasserstofffraktion mit verschiedenen Sättigungsgraden enthalten sind.

5. Verfahren zur Extraktion von Diolefinen in der Flüssigphase, die in einer Kohlenwasserstofffraktion mit verschiedenen Sättigungsgraden enthalten sind, umfassend mindestens einen Flüssig-Flüssig-Extraktionsschritt im Gegenstrom, der durch die Kohlenwasserstofffraktion gespeist wird, und durch eine Extraktionslösung, wobei die Extraktionslösung eine absorbierende Lösung, wie in einem der Ansprüche 1 bis 3 definiert, ist.

6. Verfahren nach Anspruch 5, umfassend mindestens:

a) einen Flüssig-Flüssig-Extraktionsschritt im Gegenstrom, der gespeist wird:

- an mindestens einem Zwischenpunkt durch die Kohlenwasserstofffraktion;
- am Kopf durch eine Extraktionslösung, welche aus der polaren Extraktionslösung, die aus Schritt e) stammt, und dem Reextraktionsraffinat, das aus Schritt b) stammt, besteht,
- am Boden durch eine Hilfsextraktionslösung, welche aus einer Fraktion der apolaren Hilfslösung, die aus dem Dekantierungsschritt e) stammt, und/oder einer Fraktion des Reextraktionsextrakts, der am Kopf der Extraktionssäule im Reextraktionsschritt b) abgezogen wird, besteht,

wobei bei einer Temperatur zwischen 20 und 60°C, einem Druck zwischen 0,1 und 1 MPa und einem Gewichtsverhältnis Durchsatz der Extraktionslösung/Durchsatz der Kohlenwasserstofffraktion von weniger als 1 gearbeitet wird und erzeugt werden:

- am Kopf ein Hauptraffinat, umfassend mehr als 95 % Olefin- und Paraffin-Kohlenwasserstoffe, die in der Kohlenwasserstofffraktion enthalten sind,
- und am Boden ein Hauptextrakt, umfassend mehr als 95 % Diolefin-Kohlenwasserstoffe, die in der Kohlenwasserstofffraktion enthalten sind,

b) einen Flüssig-Flüssig-Reextraktionsschritt im Gegenstrom, der gespeist wird:

- am Kopf durch den Hauptextrakt, der aus Schritt a) stammt,
- am Boden durch eine Fraktion der apolaren Hilfslösung, die aus Schritt e) stammt,

wobei bei einer Temperatur zwischen 20 und 60°C, einem Druck zwischen 0,1 und 1 MPa gearbeitet wird, wobei das Gewichtsverhältnis Durchsatz der apolaren Hilfslösungsfraktion/Durchsatz des Hauptextrakts zwischen 0,1 und 1 liegt und erzeugt werden:

- am Kopf ein Reextraktionsextrakt, umfassend mindestens 90 % Diolefin-Kohlenwasserstoffe, die in dem Hauptextrakt enthalten sind,
- und am Boden ein Reextraktionsraffinat,

c) einen Trennschritt der Diolefine, wobei mindestens eine Fraktion des Reextraktionsextrakts, der aus Schritt b) stammt, an mindestens einem Zwischenpunkt einer Destillationssäule zugeführt wird, ein Diolefin-Abfluss am Kopf der Säule abgezogen wird, und ein Trennrückstand der Diolefine am Boden der Säule abgezogen wird,
d) einen Trennschritt der Kohlenwasserstoffe, wobei das Hauptraffinat, das aus Schritt a) stammt, an mindestens einem Zwischenpunkt einer Destillationssäule zugeführt wird, ein Kohlenwasserstoff-Abfluss am Kopf der Säule abgezogen wird, und ein Trennrückstand der Kohlenwasserstoffe am Boden der Säule abgezogen wird,
e) einen Dekantierungsschritt, der durch den Trennrückstand der Diolefine, der aus Schritt c) stammt, und den Trennrückstand der Kohlenwasserstoffe, der aus Schritt d) stammt, gespeist wird, wobei die Trennung einer polaren Extraktionslösung und einer apolaren Hilfslösung gestattet wird, wobei die polare Extraktionslösung anschließend zu Schritt a) rückgeführt wird, wobei die apolare Hilfslösung anschließend zu Schritt b) und/oder Schritt a) rückgeführt wird,

wobei die in Schritt a) zugeführte Extraktionslösung eine absorbierende Lösung, wie in einem der Ansprüche 1 bis 3 definiert, ist.

7. Verfahren nach Anspruch 6, wobei die den Extraktionsschritt a) speisende Hilfsextraktionslösung aus einer Fraktion der apolaren Hilfslösung besteht, die aus Schritt e) stammt.

8. Verfahren nach einem der Ansprüche 6 bis 7, wobei die Hilfsextraktionslösung, die den Extraktionsschritt a) speist, aus einer Fraktion des Reextraktionsextrakts besteht, der aus Schritt b) stammt.

9. Verfahren nach einem der Ansprüche 5 bis 8, wobei die Kohlenwasserstofffraktion 20 bis 99,5 Gew.-% Diolefine umfasst.

**10.** Verfahren nach einem der Ansprüche 6 bis 9, wobei das Hauptraffinat mehr als 99 % Olefin- und Paraffin-Kohlenwasserstoffe umfasst, die in der Kohlenwasserstofffraktion enthalten sind.

**11.** Verfahren nach einem der Ansprüche 6 bis 10, wobei der Hauptextrakt mehr als 99 % Diolefin-Kohlenwasserstoffe umfasst, die in der Kohlenwasserstofffraktion enthalten sind.

**12.** Verfahren nach einem der Ansprüche 6 bis 11, wobei Schritt c) bei einem Druck von weniger als dem Betriebsdruck der Säule arbeitet, die in Schritt d) verwendet wird.

**13.** Verfahren nach einem der Ansprüche 6 bis 12, wobei Schritt d) bei einem Druck zwischen 0,1 und 1 MPa arbeitet.

**14.** Verfahren nach einem der Ansprüche 6 bis 13, wobei Schritt e) bei einer Temperatur zwischen 20 und 60°C und einem Druck zwischen 0,1 und 1 MPa arbeitet.

**Claims**

**1.** Absorbent solution containing as the only polar solvents a mixture of ethylene carbonate (EC) and propylene carbonate (PC) in an EC/PC mass ratio comprised between 90/10 and 60/40, saturated with apolar solvents selected from a paraffinic or cycloparaffinic hydrocarbon or a mixture of paraffinic and cycloparaffinic hydrocarbons having from 5 to 10 carbon atoms per molecule.

**2.** Absorbent solution according to claim 1 wherein the EC/PC mass ratio is comprised between 75/25 and 65/35.

**3.** Absorbent solution according to any one of claims 1 or 2, wherein said apolar solvent is a cut of paraffinic and cycloparaffinic hydrocarbons with 5 and 6 carbon atoms, and preferably cyclohexane.

**4.** Use of an absorbent solution according to any one of claims 1 to 3, in a process for the liquid-phase extraction of the diolefins contained in a hydrocarbons feedstock with various degrees of saturation.

**5.** Process for the liquid-phase extraction of the diolefins contained in a hydrocarbons feedstock with various degrees of saturation comprising at least one stage of counter-current liquid-liquid extraction fed with said hydrocarbons feedstock, and with an extraction solution, said extraction solution being an absorbent solution as defined according to one of claims 1 to 3.

**6.** Process according to claim 5 comprising at least:

a) a stage of counter-current liquid-liquid extraction fed:

- at at least one intermediate point with said hydrocarbons feedstock,
- at the top with an extraction solution constituted by the polar extraction solution obtained at stage e) and the re-extraction raffinate obtained at stage b),
- at the bottom with an auxiliary extraction solution constituted by a fraction of the auxiliary apolar solution obtained at decantation stage e) and/or by a fraction of the re-extraction extract drawn off at the top of the extraction column of the re-extraction stage b),

operating at a temperature comprised between 20 and 60°C, a pressure comprised between 0.1 and 1 MPa and a weight ratio of extraction solution flow rate/hydrocarbons feedstock flow rate less than 1 and producing:

- at the top a main raffinate, comprising more than 95% olefinic and paraffinic hydrocarbons comprised in said hydrocarbons feedstock,
- and at the bottom a main extract comprising more than 95% diolefinic hydrocarbons comprised in said hydrocarbons feedstock,

b) a stage of counter-current liquid-liquid re-extraction fed:

- at the top with said main extract obtained at stage a),
- at the bottom with a fraction of the auxiliary apolar solution obtained at stage e), operating at a temperature comprised between 20 and 60°C, a pressure comprised between 0.1 and 1 MPa, the weight ratio of auxiliary apolar solution fraction flow rate/main extract flow rate being comprised between 0.1 and 1, and producing:
- at the top a re-extraction extract comprising at least 90% diolefinic hydrocarbons comprised in said main extract,
- and at the bottom a re-extraction raffinate,

c) a stage of separation of the diolefins wherein at least one fraction of said re-extraction extract obtained at stage b) is fed at at least one intermediate point of a distillation column, a diolefins effluent is drawn off at the top of said column, and a separation residue of diolefins is drawn off at the bottom of said column,
d) a hydrocarbons separation stage wherein said main raffinate obtained at stage a) is fed at least one intermediate point of a distillation column, a hydrocarbons effluent is drawn off at the top of said column, and a separation residue of hydrocarbons is drawn off at the bottom of said column,
e) a decantation stage fed with said separation residue of diolefins obtained at stage c) and said separation residue of hydrocarbons obtained at stage d), allowing the separation of a polar extraction solution and an auxiliary apolar solution, said polar extraction solution then being recycled to stage a), said auxiliary apolar solution then being recycled to stage b) and/or stage a),

said extraction solution fed in said stage a) being an absorbent solution as defined according to one of claims 1 to 3.

7. Process according to claim 6 wherein said auxiliary extraction solution feeding said extraction stage a) is constituted by a fraction of said auxiliary apolar solution obtained at said stage e).

8. Process according to any one of claims 6 to 7 wherein said auxiliary extraction solution feeding said extraction stage a) is constituted by a fraction of said re-extraction extract obtained at stage b).

9. Process according to any one of claims 5 to 8 wherein said hydrocarbons feedstock comprises 20 to 99.5% by weight diolefins.

10. Process according to any one of claims 6 to 9 wherein said main raffinate comprises more than 99% olefinic and paraffinic hydrocarbons comprised in said hydrocarbons feedstock.

11. Process according to any one of claims 6 to 10 wherein said main extract comprises more than 99% diolefinic hydrocarbons comprised in said hydrocarbons feedstock.

12. Process according to any one of claims 6 to 11 wherein said stage c) operates at a pressure less than the operating pressure of the column used in said stage d).

13. Process according to any one of claims 6 to 12 wherein said stage d) operates at a pressure comprised between 0.1 and 1 MPa.

14. Process according to any one of claims 6 to 13 wherein said stage e) operates at a temperature comprised between 20 and 60°C and a pressure comprised between 0.1 and 1 MPa.

Figure 1

EP 3 074 371 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2036057

- US 3328480 A